# EUROPEAN PATENT APPLICATION

(11) **EP 3 520 679 A1**
(43) Date of publication of application: **07.08.2019**
(21) Application number: 18248157.2
(22) Date of filing: 28.12.2018
(51) Int. Cl.: A61B 3/10, G09B 23/30

(54) **MODEL EYE DESIGN FOR CALIBRATING IMAGING SYSTEMS AND RELATED METHODS, SYSTEMS AND DEVICES**

(30) Priority: 03.01.2018 US 201815860707
(71) Applicant: Leica Microsystems, Inc., Buffalo Grove IL 60089 (US)
(72) Inventor: Hart, Robert H., 27511 Cary, NC (US); Murnan, Andrew, 12866 Saratoga Springs, NY (US); Farmiga, Nestor O., 14618-3408 Rochester, NY (US); Saxer, Christopher, 28461 Southport, NC (US); Dhalla, Al-Hafeez Z., 27516 Chapel Hill, NC (US); Buckland, Eric L., 28601 Hickory, NC (US)
(74) Representative: m patent group

(57) **Abstract**

A model eye (405) for use with an imaging system is provided. The model eye includes a shape that mimics a shape of a real eye of a subject and focal parameters of the real eye of the subject. The model eye is positioned in the imaging system in place of the real eye to assess and/or calibrate the imaging system. Related methods, systems and devices are also provided herein.

## Description

### FIELD

The present inventive concept relates generally to imaging and, more particularly, to calibration of imaging systems and related methods, systems and devices.

### BACKGROUND

Optical coherence tomography (OCT) and, in particular, Fourier domain optical coherence tomography (FDOCT) is a standard of care in clinical ophthalmology. FDOCT systems acquire images of translucent structures rapidly and at high resolution, but have limited imaging depth due to optical constraints. A technique for obtaining an image of extended structures of the eye, suitable for computing the refractive properties of the eye and measuring axial and lateral distances of the eye has the potential to provide all of the benefits of LCI, OCT, topography, and aberrometry in one consolidated instrument. One such technique is discussed in, for example, commonly assigned U.S. Patent No. 9,119,563, the contents of which is hereby incorporated herein by reference as if set forth in its entirety. However, as is understood by those having skill in the art, these imaging systems must be calibrated before images can be obtained. Calibration of an imaging system typically requires presence of the patient, which can greatly increase the amount of time a patient is present in the clinical environment.

### SUMMARY

The present inventive concept provides a model eye for use with an imaging system, the model eye including a shape that mimics a shape of a real eye of a subject; and focal parameters of the real eye of the subject. The model eye is positioned in the imaging system in place of the real eye to assess and/or calibrate the imaging system.

In further embodiments, the model eye may include a lens and the lens may include an elongated body having a first end and a second end; a first end cap on the first end of the elongated body; and a second end cap on the second end of the elongated body.

In still further embodiments, the elongated body may include fused silica; and the first and second end caps may be BK7 glass.

In some embodiments, one of the first and second end caps may include an etched pattern thereon that assists in proper orientation of the lens during operation of the imaging system.

In further embodiments, the model eye may include a lens and the lens may include an elongated body having a first and a second end; and a rounded end cap on one of the first and second ends of the elongated body.

In still further embodiments, the elongated body and the rounded end cap may be BK7 glass.

In some embodiments, one of the elongated body and rounded end cap may include an etched pattern thereon that assists in proper orientation of the lens during operation of the imaging system.

In further embodiments, the model eye may include a lens is configured to be received by a mechanical cell configured to be positioned in a mount associated with the imaging system.

In still further embodiments, the imaging system may include an optical coherence tomography (OCT) imaging system.

The present inventive concept further provides methods for calibrating an imaging system, the methods include positioning a model eye in the imaging system in place of a real eye; and calibrating the imaging system using the model eye. The model eye may have a shape that mimics a shape of the real eye of a subject and focal parameters of the real eye of the subject.

In further embodiments, the model eye may include a lens and positioning the model eye may further include positioning the lens in a mechanical cell; and positioning the mechanical cell in the imaging system that is configured to receive the mechanical cell.

In still further embodiments, positioning the mechanical cell may further include positioning the mechanical cell in a mechanical mount under a portion of the imaging system in place of a subject being imaged.

In some embodiments, the imaging system may be an optical coherence tomography (OCT) imaging system.

Still further embodiments of the present inventive concept provide a mechanical cell including a cell portion configured to receive an elongated lens of a model eye; and a clamp ring configured to be positioned on an open end of the cell portion to stabilize the elongated lens when positioned in the cell portion.

In some embodiments, the mechanical cell may be configured to be positioned in a target holder positioned under an imaging system.

In further embodiments, the model eye may include a shape that mimics a shape of a real eye of a subject; and focal parameters of the real eye of the subject. The model eye may be positioned in an imaging system in place of the real eye to assess and/or calibrate the imaging system.

In still further embodiments, the imaging system may include an optical coherence tomography (OCT) imaging system.

The present inventive concept further provides a system for calibrating an imaging system, the system including a target holder positioned under the imaging system; a mechanical cell configured to be received by the target holder; and a model eye configured to be positioned in the mechanical cell. The model eye includes a shape that mimics a shape of a real eye of a subject; and focal parameters of the real eye of the subject. The model eye is positioned in the imaging system in place of the real eye to assess and/or calibrate the imaging system.

In further embodiments, the imaging system comprises an optical coherence tomography (OCT) imaging system.

### BRIEF DESCRIPTION OF THE DRAWINGS

Fig. 1 is a block diagram illustrating an example OCT system.
Fig. 2 is a block diagram illustrating an example OCT retinal imaging system.
Fig. 3 is a block diagram illustrating an example OCT cornea imaging system.
Fig. 4A is a table illustrating example Bioptigen and Optical parameters in accordance with some embodiments of the present inventive concept.
Fig. 4B is a diagram of a reference eye model in accordance with some embodiments of the present inventive concept.
Fig. 5 is a diagram of a reference eye and a Volk surgical lens in accordance with some embodiments of the present inventive concept.
Fig. 6A is a model eye lens including an elongated portion (plug) and two end caps in accordance with some embodiments of the present inventive concept.
Fig. 6B is a model eye lens including an elongated portion and a curved end cap in accordance with some embodiments of the present inventive concept.
Fig. 6C is a table comparing embodiments of the model eye lens and the human eye in accordance with some embodiments of the present inventive concept.
Fig. 7 is a diagram illustrating optical performance of the eye models in Figs. 6A and 6B at 555 nm in accordance with some embodiments of the present inventive concept.
Figs. 9A through 9F are diagrams illustrating details of a model eye lens of Fig. 6B in accordance with some embodiments of the present inventive concept.
Figs. 10A through 10C are diagrams illustrating details of the mechanical cell in accordance with some embodiments of the present inventive concept.
Fig. 11 is a diagram of a mechanical cell and clamp ring in accordance with some embodiments of the present inventive concept.
Fig. 12 is a diagram of an assembled mechanical cell in accordance with some embodiments of the present inventive concept.
Fig. 13 is a diagram of a target holder and mechanical cell in accordance with some embodiments of the present inventive concept.
Fig. 14 is a flowchart illustrating operations for calibrating an imaging system in accordance with some embodiments of the present inventive concept.
Fig. 15 is a block diagram illustrating a data processing system that can be used in accordance with some embodiments of the present inventive concept.

### DETAILED DESCRIPTION

The present inventive concept will be described more fully hereinafter with reference to the accompanying figures, in which embodiments of the inventive concept are shown. This inventive concept may, however, be embodied in many alternate forms and should not be construed as limited to the embodiments set forth herein.

Accordingly, while the inventive concept is susceptible to various modifications and alternative forms, specific embodiments thereof are shown by way of example in the drawings and will herein be described in detail. It should be understood, however, that there is no intent to limit the inventive concept to the particular forms disclosed, but on the contrary, the inventive concept is to cover all modifications, equivalents, and alternatives falling within the scope of the inventive concept as defined by the claims. Like numbers refer to like elements throughout the description of the figures.

The terminology used herein is for the purpose of describing particular embodiments only and is not intended to be limiting of the inventive concept. As used herein, the singular forms "a", "an" and "the" are intended to include the plural forms as well, unless the context clearly indicates otherwise. It will be further understood that the terms "comprises", "comprising," "includes" and/or "including" when used in this specification, specify the presence of stated features, integers, steps, operations, elements, and/or components, but do not preclude the presence or addition of one or more other features, integers, steps, operations, elements, components, and/or groups thereof. Moreover, when an element is referred to as being "responsive" or "connected" to another element, it can be directly responsive or connected to the other element, or intervening elements may be present. In contrast, when an element is referred to as being "directly responsive" or "directly connected" to another element, there are no intervening elements present. As used herein the term "and/or" includes any and all combinations of one or more of the associated listed items and may be abbreviated as "/".

Unless otherwise defined, all terms (including technical and scientific terms) used herein have the same meaning as commonly understood by one of ordinary skill in the art to which this inventive concept belongs. It will be further understood that terms used herein should be interpreted as having a meaning that is consistent with their meaning in the context of this specification and the relevant art and will not be interpreted in an idealized or overly formal sense unless expressly so defined herein.

It will be understood that, although the terms first, second, etc. may be used herein to describe various elements, these elements should not be limited by these terms. These terms are only used to distinguish one element from another. For example, a first element could be termed a second element, and, similarly, a second element could be termed a first element without departing from the teachings of the disclosure. Although some of the diagrams include arrows on communication paths to show a primary direction of communication, it is to be understood that communication may occur in the opposite direction to the depicted arrows.

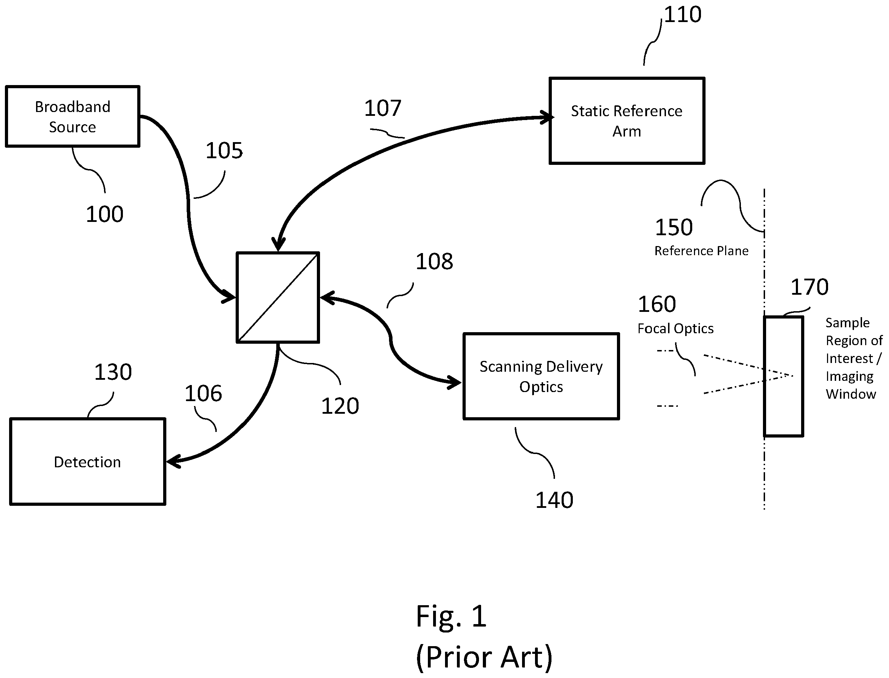

As discussed above, optical coherence tomography (OCT) systems are being used to image all parts of the eye including the cornea, retina and anything in between. Before these systems can be used to image an eye, or any other sample, the system typically needs to be calibrated. This calibration process typically involves the patient being present for some or all of the calibration, which may substantially increase the patient's time in the clinical environment. Thus, according to some embodiments of the present inventive concept, a model eye is provided for use in calibrating the system so that the patient does not necessarily have to be present during this portion of the process. As will be discussed further herein with respect to Figs. 1 through 15, a model eye according to embodiments of the present inventive concept mimics both the shape and focal parameters of a real eye, for example, a human eye.

Referring now to Figs. 1 through 3, Fourier Domain Optical Coherence Tomography (FDOCT) systems that may be used in combination with the model eye discussed herein will be discussed. It will be understood that these systems are provided for example only and other systems including different elements may be used without departing from the scope of the present inventive concept. Referring first to Fig. 1, a block diagram illustrating an FDOCT system will be discussed. As illustrated in Fig. 1, the system includes a broadband source 100, a reference arm 110 and a sample arm 140 coupled to each other by a beamsplitter 120. The beamsplitter 120 may be, for example, a fiber optic coupler or a bulk or micro-optic coupler without departing from the scope of the present invention. The beamsplitter 120 may provide from about a 50/50 to about a 90/10 split ratio. As further illustrated in Fig. 1, the beamsplitter 120 is also coupled to a wavelength or frequency sampled detection module 130 over a detection path 106 that may be provided by an optical fiber.

As further illustrated in Fig. 1, the source 100 is coupled to the beamsplitter 120 by a source path 105. The source 100 may be, for example, a superluminescent light emitting diode (SLED) or tunable source. The reference arm 110 is coupled to the beamsplitter over a reference arm path 107. Similarly, the sample arm 140 is coupled to the beamsplitter 120 over the sample arm path 108. The source path 105, the reference arm path 107 and the sample arm path 108 may all be provided by optical fiber.

As further illustrated in Fig. 1, the sample arm 140 may include scanning delivery optics and focal optics 160. Also illustrated in Fig. 1 is the reference plane 150 and a representation of an OCT imaging window 170 (sample region of interest).

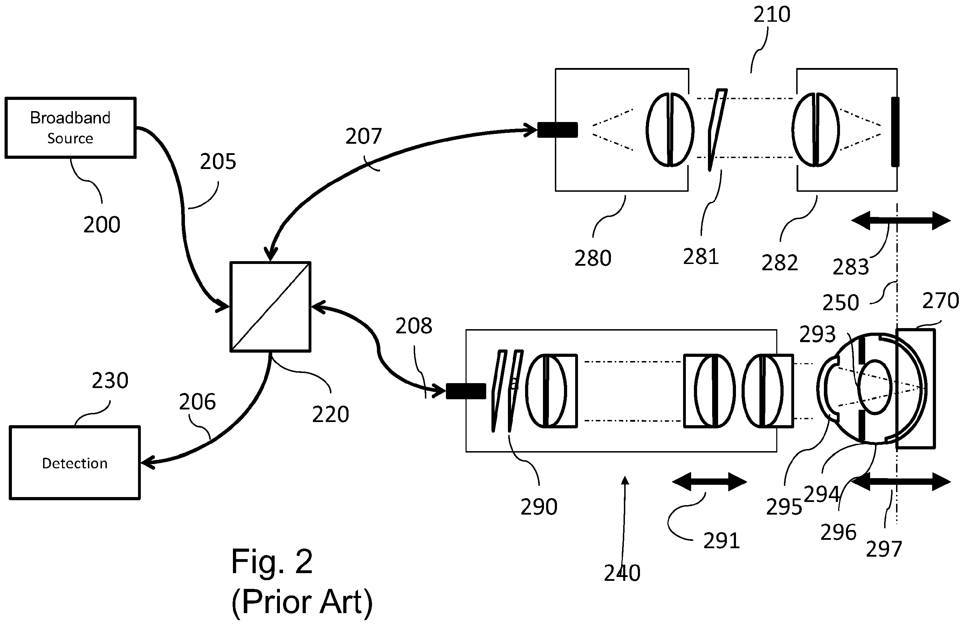

Referring now to Fig. 2, a block diagram of an FDOCT retinal imaging system will be discussed. As illustrated in Fig. 2, in an FDOCT retinal imaging system, the reference arm 210 may further include a collimator assembly 280, a variable attenuator 281 that can be neutral density or variable aperture, a mirror assembly 282, a reference arm variable path length adjustment 283 and a path length matching position 250, i.e. optical path length reference to sample. As further illustrated, the sample arm 240 may include a dual-axis scanner assembly 290 and a variable focus objective lens 291.

The sample in Fig. 2 is an eye including a cornea 295, iris/pupil 294, ocular lens 293 and retina 296. A representation of an OCT imaging window 270 is illustrated near the retina 296. The retinal imaging system relies in the optics of the subject eye, notably cornea 295 and ocular lens 293, to image the posterior structures of the eye. As further illustrated, embodiments of Fig. 2 include objective lens variable focus 297.

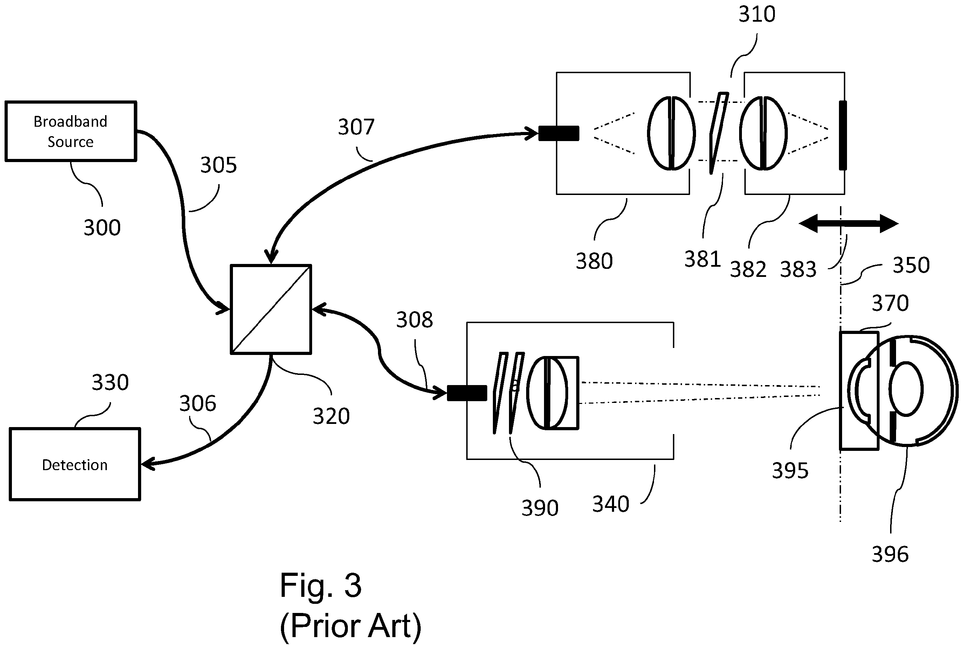

Referring now to Fig. 3, a block diagram illustrating a FDOCT corneal imaging system will be discussed. As illustrated therein, the system of Fig. 3 is very similar to the system of Fig. 2. However, the objective lens variable focus need not be included, and is not included in Fig. 3. The anterior imaging system of Fig. 3 images the anterior structures directly, without reliance on the optics of the subject to focus on the anterior structures.

As discussed above, embodiments of the present inventive concepts are not limited to OCT or FDOCT systems, any imaging system can be used without departing from the scope of the present inventive concept discussed herein.

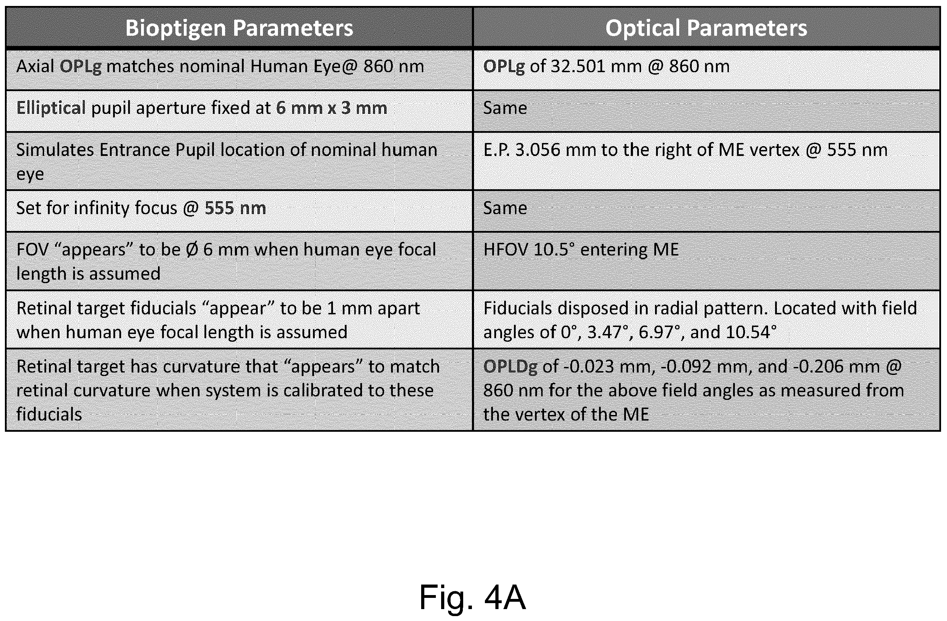

Before creating a model eye for use in both assessing and calibrating an imaging system, parameters may be established for the particular model should be capable. Fig. 4A illustrates an example listing of Bioptigen's parameters for embodiments of the model eye as well as optical parameters for the model eye.

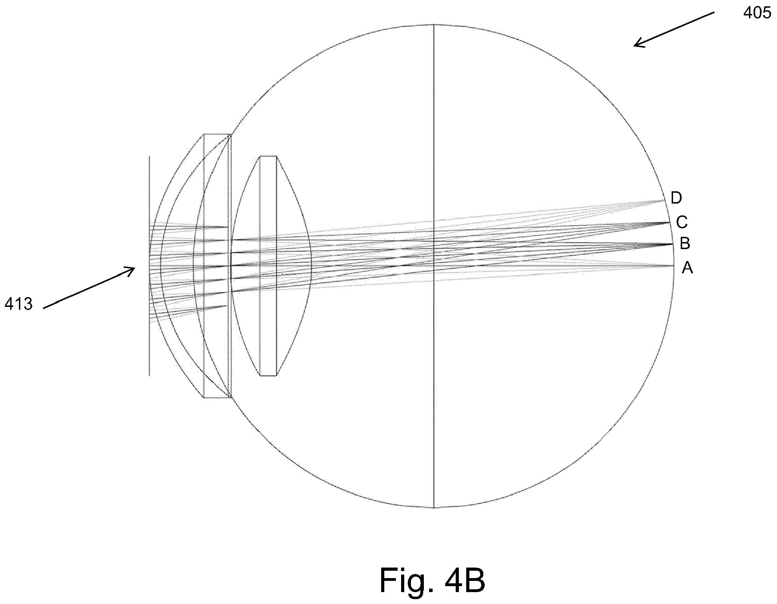

Referring now to Fig. 4B, a reference eye will be discussed in accordance with embodiments of the present inventive concept. A reference surface of the eye (vertex of the corneal plane is designated by reference numeral 413. The chief rays having a retinal height of 0 mm is represented by the grouping labeled A; the chief rays having a retinal height of 1 mm is represented by the grouping labeled B; chief rays having a retinal height of 2 mm is represented by the grouping labeled C and chief rays having a retinal height of 3 mm is represented by the grouping labeled D. Some details of the human eye model 405 illustrated in Fig. 4B are as follows. The entrance Pupil (E.P.) is located 3.056 mm inside the eye from reference surface 413 at 555 nm. An E.P. is set to Ø at 4mm and a radius of curvature of the retina is 11mm. HFOV angles 0°, 3.47°, 6.97° and 10.54° correspond to chief ray retinal heights 0 mm (A), 1 mm(B), 2 mm (C) and 3 mm(D), respectively. Axial OPLg is 32.501 mm at 860 nm; EFL is 16.714 mm at 860 nm and it is emmetropic at 555 nm.

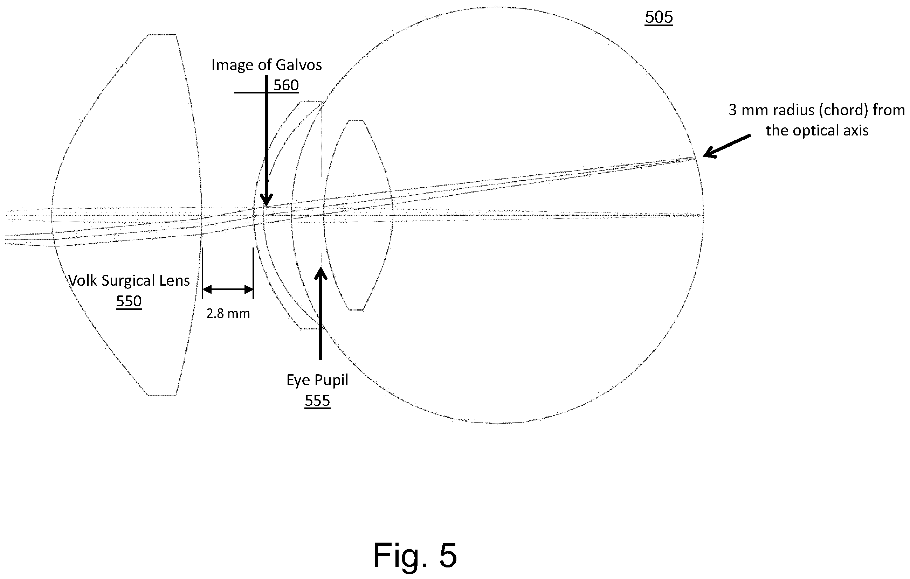

Referring now to Fig. 5, the model eye in combination with a Volk Surgical Lens will be discussed. As illustrated in Fig. 5, there is a 2.8 mm spacing between the volk surgical lens 550 and the image of the galvos 560 of the model eye 505. Also shown in Fig. 5 are the eye pupil 555 and a 3mm radius (chord) from the optical axis passing therethrough. In this example, there is a collimated SMI beam of less than Ø 1 mm entering HE/ME with input beam zoom (IBZ) set to low numerical aperture (NA) condition.

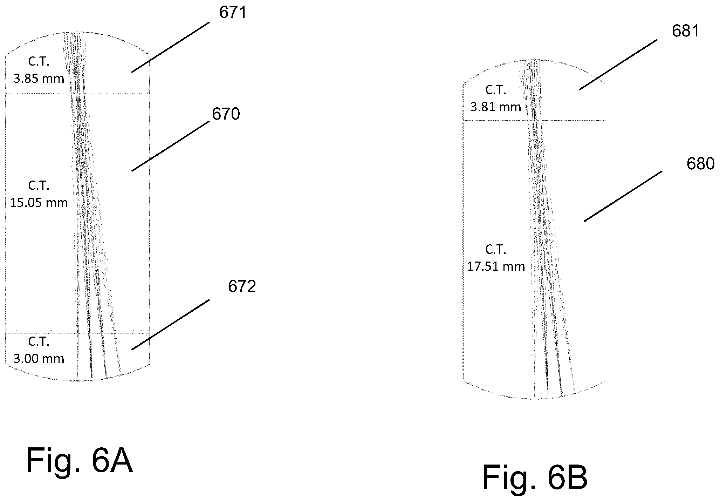

Model eyes in accordance with some embodiments of the present inventive concept will now be discussed with respect to Figs. 6A through 6C. As discussed above, model eyes in accordance with some embodiments discussed herein may be used to assess and/or calibrate an imaging system without having the patient present in the clinical environment. Thus, model eyes in accordance with embodiments herein have a shape that mimics a shape of a real eye of a subject and focal parameters of the real eye of the subject.

Referring first embodiments illustrated in Fig. 6A, in some embodiments of the present inventive concept, the model eye includes a lens having an elongated body 670 having first and second ends, a first end cap 671 on the first end of the elongated body 670 and a second end cap 672 on the second end of the elongated body 670. In some embodiments, the elongated body 670 includes fused silica (FS) and the first and second end caps include BK7 glass. These materials are provided as examples only and, therefore, it will be understood that embodiments of the present inventive concept are not limited thereto.

As will be discussed further below, one of the first and second end caps or the elongated body may include an etched pattern thereon that assists in proper orientation of the lens during operation of the imaging system. In other words, there may be hash marks on overlapping axes that facilitate alignment and orientation of the sample within the imaging system.

Referring now to embodiments illustrated in Fig. 6B, the model eye lens includes an elongated body 680 having a first and a second end and a rounded end cap 681 on one of the first and second ends of the elongated body 680. In these embodiments, the elongated body and the rounded end cap both include the same material, for example, BK7 glass. Again, as will be discussed further below, one of the elongated body and rounded end cap may include an etched pattern thereon that assists in proper orientation of the lens during operation of the imaging system.

As will be discussed below, the lens is configured to be received by a mechanical cell configured to be positioned in a mount associated with the imaging system. The cell and the mount allow the lens to be integrated with the imaging system for calibration. In some embodiments, the imaging system may be an OCT imaging system, however, embodiments of the present inventive concept are not limited thereto.

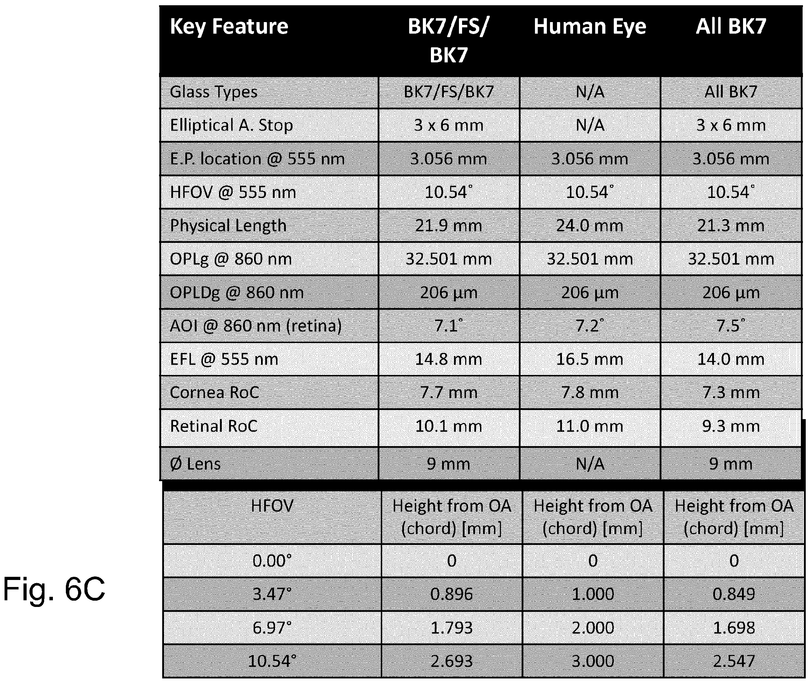

Referring now to Fig. 6C, embodiments of the model eye lens having two BK7 ends caps (Fig. 6A) and including all BK7 glass (Fig. 6B) are compared to an actual human eye sample. The various parameters are set out in detail in Fig. 6C.

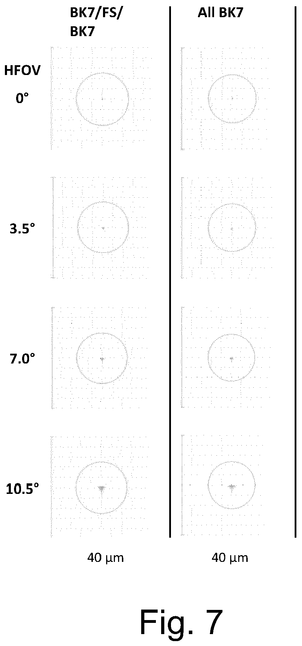

Referring now to Fig. 7, optical performance of the model eye lens in accordance with various embodiments of the present inventive concept will be discussed. The optical performance at 555 nm and a low NA are illustrated for embodiments in Fig. 6A (BK7/FS/BK7) and Fig. 6B (all BK7) for Half Field of View angles or HFOVs of 0°; 3.5°; 7° and 10.5°. In all illustrated embodiments, the design produces diffraction limited optical performance for both wavelength regions of 555nm and near infrared centered at 860nm.

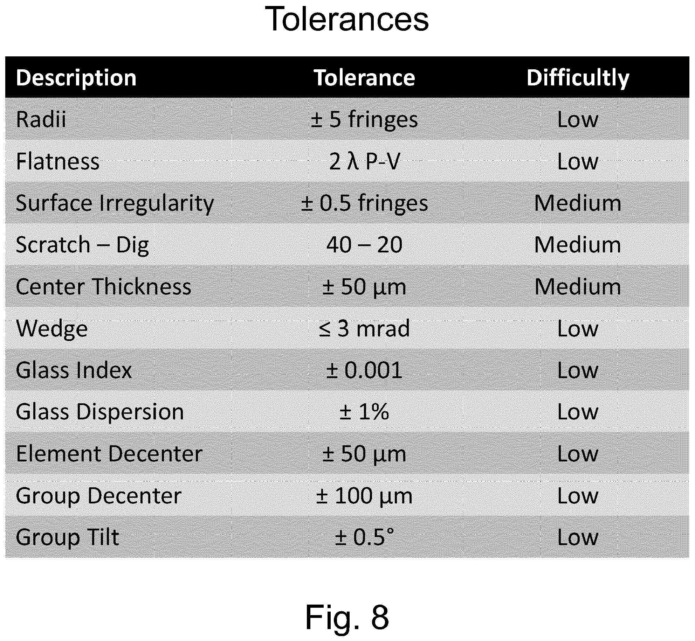

Thermal analysis shows no change over a temperature range of 15 degrees to 25 degrees. In a human eye, Group Delay Dispersion (GDD) of approximately 957 fs2 and a physical length of 24.000 mm yield a GVD of -39.9 fs2/mm. In a BK7/FS/BK7 model eye (ME) (Fig. 6A) a GDD of approximately 741 fs2 and a physical length of 21.902 mm yield a GVD of -33.8 fs2/mm. In the all BK7 ME (Fig. 6B) a GDD of approximately 835 fs2 and a physical length of 21.318 mm yield a GVD of -39.2 fs2/mm. Fig. 8 is a table illustrating various tolerances of the model eye in accordance with embodiments of the present inventive concept.

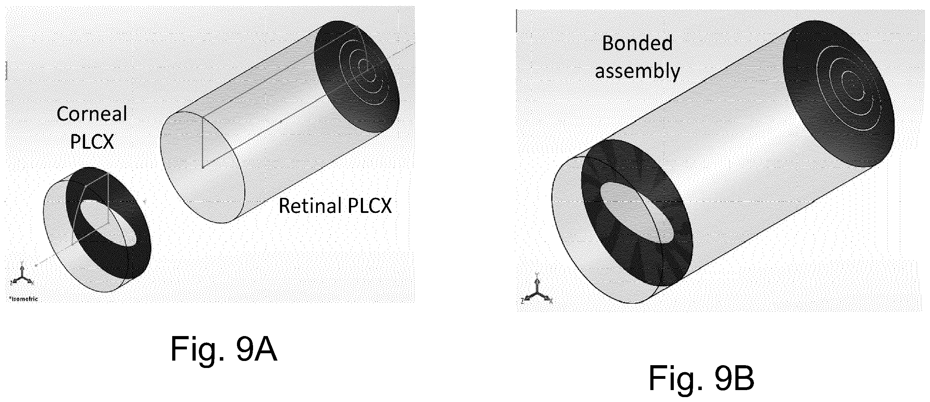

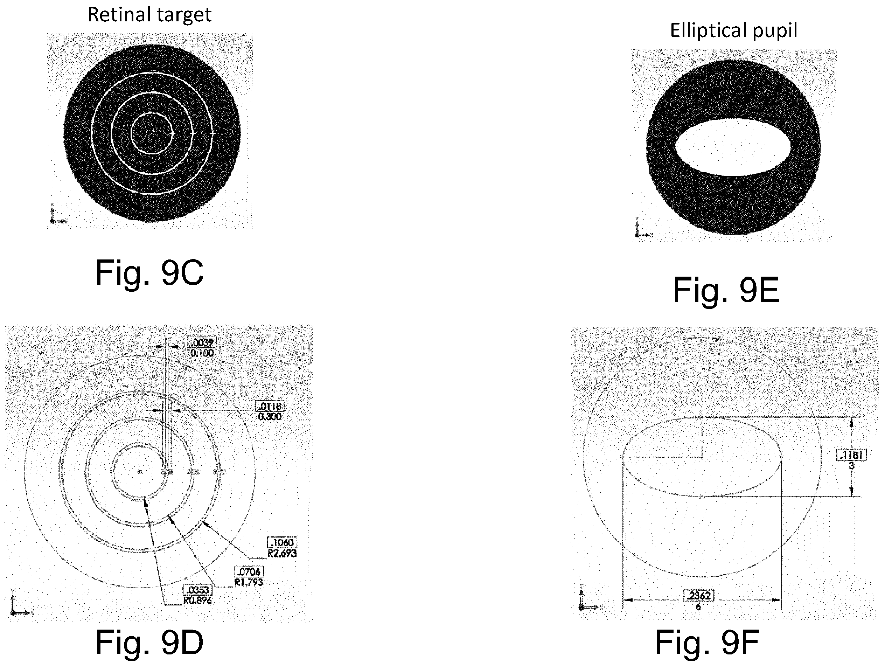

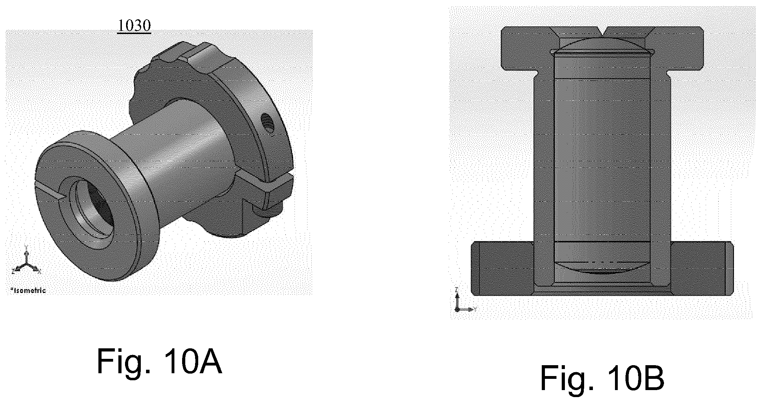

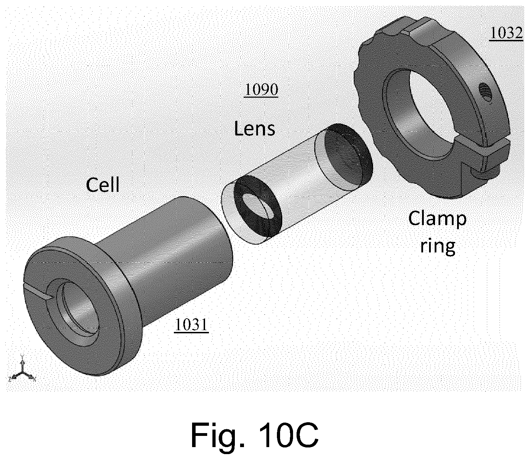

Referring now to Figs. 9A through 9E, embodiments of the model eye lens illustrated in Fig. 6B will be discussed in more detail. Referring first to Fig. 9A, the model eye lens comprises an elongated body (Retinal PLCX) and a curved end cap (Corneal PLCX). The two portions of the model eye lens are shown separately in Fig. 9A and bonded together in Fig. 9B. As discussed above, some embodiments of the present inventive concept include a target etched on the lens to facilitate alignment and orientation. Retinal targets in accordance with some embodiments of the present inventive concept are illustrated in Figs. 9C and 9D. As illustrated the targets may have various circles and ticks indicating measured distances. The targets illustrated in Figs. 9C and 9D are provided for example only and, thus, embodiments are not limited to this configuration. In the model eye lens, the pupil is represented as an ellipse as illustrated in, for example, Figs. 9E and 9F. The elliptical shape of the model eye pupil provides simultaneous alignment and orientation information as well as simultaneous imaging properties which mimic both a constricted (Miotic) and dilated (Mydriatic) pupil. As discussed above, the model eye lens in accordance with embodiments of the present inventive concept is positioned in a mechanical cell for use by the imaging system. Referring now to Figs. 10A through 10C, a mechanical cell in accordance with some embodiments of the present inventive will be discussed. Referring first to Fig. 10A, the mechanical cell including a cell portion for receiving the model eye lens and a clamp ring positioned on an open end of the cell portion to stabilize the model eye lens when positioned in the cell portion are shown together. Fig. 10B is a cross section of the mechanical cell having the model eye lens therein. Fig. 10C is an exploded view of the mechanical cell 1030 including the cell portion 1031, the model eye lens 1090 and the clamp ring 1032.

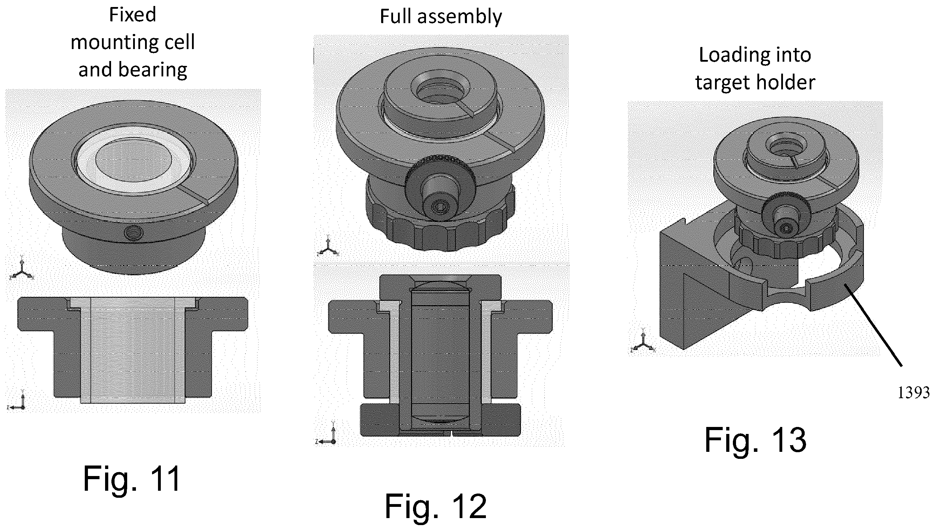

For use in the imaging system, the mechanical cell 1030 (Fig. 10A) is positioned in a target holder beneath the imaging system. A fully assembled mechanical cell including the model eye lens and positioned in a target holder in accordance with some embodiments of the present inventive concept will now be discussed with respect to Figs. 11 through 13. Referring first to Fig. 11, a cross section of a fixed mounting cell and bearing is illustrated. A fully assembled cell is shown in Fig. 12 and a mechanical cell being loaded into a target holder 1393 is shown in Fig. 13.

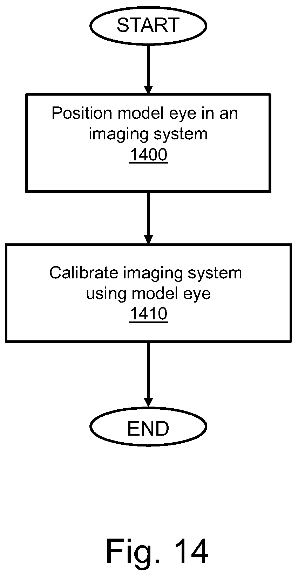

Referring now to the flowchart of Fig. 14, operations for calibrating an imaging system will be discussed. Operations begin at block 1400 by positioning a model eye lens in the imaging system in place of a human eye. As discussed above, to be placed in the imaging system, the model eye is placed in a mechanical cell and the mechanical cell is placed in target holder. However, it will be understood that embodiments of the present inventive concept are not limited to this configuration. Other methods of positioning the model eye beneath the imaging system may be used without departing from the scope of the present inventive concept.

Once the model eye lens is positioned in the imaging system, the imaging system may be calibrated and/or assessed using the model eye lens (block 1410). Thus, the imaging system may be calibrated without the patient's presence in the clinical environment, therefore, reducing the amount of time a patient must be present in the clinical environment.

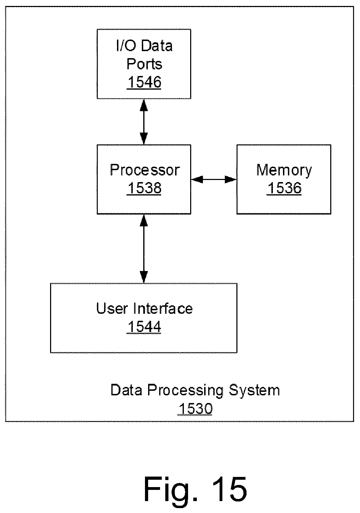

Exemplary embodiments of a data processing system 1530 configured in accordance with embodiments of the present inventive concept will be discussed with respect to Figure 15. The data processing system may be included as part of the imaging system discussed herein. The data processing system 1530 may include a user interface 1544, including, for example, input device(s) such as a keyboard or keypad, a display, a speaker and/or microphone, and a memory 1536 that communicate with a processor 1538. The data processing system 1530 may further include I/O data port(s) 1546 that also communicates with the processor 1538. The I/O data ports 1546 can be used to transfer information between the data processing system 1530 and another computer system or a network using, for example, an Internet Protocol (IP) connection. These components may be conventional components such as those used in many conventional data processing systems, which may be configured to operate as described herein.

Example embodiments are described above with reference to block diagrams and/or flowchart illustrations of methods, devices, systems and/or computer program products. It is understood that a block of the block diagrams and/or flowchart illustrations, and combinations of blocks in the block diagrams and/or flowchart illustrations, can be implemented by computer program instructions. These computer program instructions may be provided to a processor of a general purpose computer, special purpose computer, and/or other programmable data processing apparatus to produce a machine, such that the instructions, which execute via the processor of the computer and/or other programmable data processing apparatus, create means (functionality) and/or structure for implementing the functions/acts specified in the block diagrams and/or flowchart block or blocks.

These computer program instructions may also be stored in a computer-readable memory that can direct a computer or other programmable data processing apparatus to function in a particular manner, such that the instructions stored in the computer-readable memory produce an article of manufacture including instructions which implement the functions/acts specified in the block diagrams and/or flowchart block or blocks.

The computer program instructions may also be loaded onto a computer or other programmable data processing apparatus to cause a series of operational steps to be performed on the computer or other programmable apparatus to produce a computer-implemented process such that the instructions which execute on the computer or other programmable apparatus provide steps for implementing the functions/acts specified in the block diagrams and/or flowchart block or blocks.

Accordingly, example embodiments may be implemented in hardware and/or in software (including firmware, resident software, micro-code, etc.). Furthermore, example embodiments may take the form of a computer program product on a computer-usable or computer-readable storage medium having computer-usable or computer-readable program code embodied in the medium for use by or in connection with an instruction execution system. In the context of this document, a computer-usable or computer-readable medium may be any medium that can contain, store, communicate, propagate, or transport the program for use by or in connection with the instruction execution system, apparatus, or device.

The computer-usable or computer-readable medium may be, for example but not limited to, an electronic, magnetic, optical, electromagnetic, infrared, or semiconductor system, apparatus, device, or propagation medium. More specific examples (a non-exhaustive list) of the computer-readable medium would include the following: an electrical connection having one or more wires, a portable computer diskette, a random access memory (RAM), a read-only memory (ROM), an erasable programmable read-only memory (EPROM or Flash memory), an optical fiber, and a portable compact disc read-only memory (CD-ROM). Note that the computer-usable or computer-readable medium could even be paper or another suitable medium upon which the program is printed, as the program can be electronically captured, via, for instance, optical scanning of the paper or other medium, then compiled, interpreted, or otherwise processed in a suitable manner, if necessary, and then stored in a computer memory.

Computer program code for carrying out operations of data processing systems discussed herein may be written in a high-level programming language, such as Java, AJAX (Asynchronous JavaScript), C, and/or C++, for development convenience. In addition, computer program code for carrying out operations of example embodiments may also be written in other programming languages, such as, but not limited to, interpreted languages. Some modules or routines may be written in assembly language or even micro-code to enhance performance and/or memory usage. However, embodiments are not limited to a particular programming language. It will be further appreciated that the functionality of any or all of the program modules may also be implemented using discrete hardware components, one or more application specific integrated circuits (ASICs), or a field programmable gate array (FPGA), or a programmed digital signal processor, a programmed logic controller (PLC), microcontroller or graphics processing unit.

It should also be noted that in some alternate implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved. Moreover, the functionality of a given block of the flowcharts and/or block diagrams may be separated into multiple blocks and/or the functionality of two or more blocks of the flowcharts and/or block diagrams may be at least partially integrated.

In the drawings and specification, there have been disclosed exemplary embodiments of the inventive concept. However, many variations and modifications can be made to these embodiments without substantially departing from the principles of the present inventive concept. Accordingly, although specific terms are used, they are used in a generic and descriptive sense only and not for purposes of limitation, the scope of the inventive concept being defined by the following claims.

## Claims

1. A model eye (405; 505) for use with an imaging system, the model eye comprising:
a shape that mimics a shape of a real eye of a subject; and
focal parameters of the real eye of the subject,
wherein the model eye is positioned in the imaging system in place of the real eye to assess and/or calibrate the imaging system.

2. The model eye of Claim 1, wherein the model eye comprises a lens and wherein the lens comprises:
an elongated body (670) having a first end and a second end;
a first end cap (671) on the first end of the elongated body; and
a second end cap (672) on the second end of the elongated body.

3. The model eye of Claim 2:
wherein the elongated body (670) comprises fused silica; and
wherein the first and second end caps comprise BK7 glass.

4. The model eye of Claim 2 or Claim 3, wherein one of the first and second end caps comprise an etched pattern thereon that assists in proper orientation of the lens during operation of the imaging system.

5. The model eye of Claim 1, wherein the model eye comprises a lens and wherein the lens comprises:
an elongated body (680) having a first and a second end; and
a rounded end cap (681) on one of the first and second ends of the elongated body (680).

6. The model eye of Claim 5, wherein the elongated body (680) and the rounded end cap comprise BK7 glass.

7. The model eye of Claim 5 or Claim 6, wherein one of the elongated body and rounded end cap comprise an etched pattern thereon that assists in proper orientation of the lens during operation of the imaging system.

8. The model eye of any one of the preceding Claims:
wherein the model eye comprises a lens; and
wherein the lens is configured to be received by a mechanical cell configured to be positioned in a mount associated with the imaging system,wherein the imaging system particularly comprises an optical coherence tomography (OCT) imaging system.

9. A method for calibrating an imaging system, the method comprising:
positioning a model eye (405; 505) in the imaging system in place of a real eye; and
calibrating the imaging system using the model eye, wherein the model eye has a shape that mimics a shape of the real eye of a subject and focal parameters of the real eye of the subject.

10. The method of Claim 9, wherein the model eye comprises a lens (1090) and wherein positioning the model eye further comprises:
positioning the lens in a mechanical cell (1030); and
positioning the mechanical cell in the imaging system that is configured to receive the mechanical cell.

11. The method of Claim 10, wherein positioning the mechanical cell further comprises positioning the mechanical cell in a mechanical mount under a portion of the imaging system in place of a subject being imaged,wherein the imaging system particularly is an optical coherence tomography (OCT) imaging system.

12. A mechanical cell (1030) comprising:
a cell portion (1031) configured to receive an elongated lens (1090) of a model eye; and
a clamp ring (1032) configured to be positioned on an open end of the cell portion (1031) to stabilize the elongated lens (1090) when positioned in the cell portion (1031).

13. The mechanical cell of Claim 12, wherein the mechanical cell is configured to be positioned in a target holder (1393) positioned under an imaging system.

14. The mechanical cell of Claim 13 or 14, wherein the model eye comprises:
a shape that mimics a shape of a real eye of a subject; and
focal parameters of the real eye of the subject,
wherein the model eye is positioned in an imaging system in place of the real eye to assess and/or calibrate the imaging system, wherein the imaging system particularly comprises an optical coherence tomography (OCT) imaging system.

15. A system for calibrating an imaging system, the system comprising:
a target holder (1393) positioned under the imaging system;
a mechanical cell (1030) configured to be received by the target holder; and
a model eye (405; 505) configured to be positioned in the mechanical cell, the model eye comprising:
a shape that mimics a shape of a real eye of a subject; and
focal parameters of the real eye of the subject,
wherein the model eye is positioned in the imaging system in place of the real eye to assess and/or calibrate the imaging system, wherein the imaging system particularly comprises an optical coherence tomography (OCT) imaging system.
